(19) Europäisches Patentamt
European Patent Office
Office européen des brevets

(11) Veröffentlichungsnummer: **0 066 771**
**B1**

(12) **EUROPÄISCHE PATENTSCHRIFT**

(45) Veröffentlichungstag der Patentschrift: **03.10.84**

(21) Anmeldenummer: **82104536.6**

(22) Anmeldetag: **25.05.82**

(51) Int. Cl.³: **C 07 C 33/50**, C 07 C 33/48, C 07 C 43/23, A 01 N 31/04 // C07C29/62

(54) **1-Iod-1-propin-3-ole, Verfahren zu ihrer Herstellung sowie ihre Verwendung als Pflanzenschutzmittel.**

(30) Priorität: **04.06.81 DE 3122177**

(43) Veröffentlichungstag der Anmeldung: **15.12.82 Patentblatt 82/50**

(45) Bekanntmachung des Hinweises auf die Patenterteilung: **03.10.84 Patentblatt 84/40**

(84) Benannte Vertragsstaaten: **BE CH DE FR GB IT LI NL**

(56) Entgegenhaltungen:
**EP - A - 0 002 476**
**EP - A - 0 002 679**
**CH - A - 601 151**
**FR - A - 913 365**
**FR - A - 1 474 706**
**GB - A - 1 083 936**
**US - A - 1 841 768**
**US - A - 2 989 568**
**US - A - 3 506 682**

(73) Patentinhaber: **BAYER AG, Konzernverwaltung RP Patentabteilung, D-5090 Leverkusen 1 Bayerwerk (DE)**

(72) Erfinder: **Jäger, Gerhard, Dr., Gellertstrasse 18, D-5090 Leverkusen 1 (DE)**
Erfinder: **Brandes, Wilhelm, Dr., Eichendorffstrasse 3, D-5653 Leichlingen (DE)**
Erfinder: **Frohberger, Paul-Ernst, Dr., Willi-Baumeister-Strasse 5, D-5090 Leverkusen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft neue 1-Iod-1-propin-3-ole, ein Verfahren zu ihrer Herstellung sowie ihre Verwendung als Pflanzenschutzmittel.

Es ist bereits bekannt geworden, dass bestimmte Diarylalkinole, beispielsweise 3-(4-Biphenyl-1-yl)-3-(3-chlorphenyl)-1-iod-1-propin-3-ol, 3-(2-Fluorphenyl)-1-iod-3-phenyl-1-propin-3-ol oder 3,3-Diphenyl-1-iod-1-propin-3-ol fungizide Eigenschaften aufweisen (DE-OS Nr. 2756031).

Weiterhin sind Verbindungen mit einem Hydroxypropinylrest, wie z.B. Methylethylacetylencarbinol, als Insektizide bekannt (vgl. FR-PS Nr. 913365).

Deren Wirksamkeit ist jedoch insbesondere bei niedrigen Aufwandmengen und -konzentrationen nicht immer ganz befriedigend.

Es wurden neue 1-Iod-1-propin-3-ole der Formel I,

$$I-C\equiv C-\underset{R^2}{\overset{R^1}{C}}-OH \qquad (I)$$

in welcher

$R^1$ für gegebenenfalls durch Azolyl, Halogen, Halogenalkoxy und Halogenalkylthio substituiertes Aryl, und

$R^2$ für Alkyl oder Cycloalkyl steht,

gefunden.

Sie weisen starke fungizide Eigenschaften auf.

Weiterhin wurde gefunden, dass man die 1-Iod-1-propin-3-ole der Formel

$$I-C\equiv C-\underset{R^2}{\overset{R^1}{C}}-OH \qquad (I)$$

in welcher

$R^1$ für gegebenenfalls durch Azolyl, Halogen, Halogenalkoxy, und Halogenalkylthio substituiertes Aryl, und

$R^2$ für Alkyl oder Cycloalkyl steht,

erhält, wenn man 1-Propin-3-ole der Formel II

$$H-C\equiv C-\underset{R^2}{\overset{R^1}{C}}-OH \qquad (II)$$

in welcher

$R^1$ und $R^2$ die bei Formel I angegebene Bedeutung haben,

mit Iod in Gegenwart einer basischen Verbindung und gegebenenfalls in Gegenwart eines Verdünnungsmittels umsetzt.

Überraschenderweise zeigen die erfindungsgemässen 1-Iod-1-propin-3-ole eine erheblich höhere fungizide Wirkung als die bekannten Diarylalkinole. Die erfindungsgemässen Wirkstoffe stellen eine Bereicherung der Technik dar.

Die erfindungsgemässen 1-Iod-1propin-3-ole sind durch Formel I allgemein definiert. In dieser Formel stehen vorzugsweise

$R^1$ für gegebenenfalls durch Reste 1,2,4-Triazol-1-yl und Imidazol-1-yl, Fluor, Chlor, Brom, Iod, Halogenalkoxy und Halogenalkylthio mit jeweils 1 oder 2 C-Atomen und 1 bis 5 Halogenatomen substituiertes Aryl mit 6 bis 10 C-Atomen, und

$R^2$ für geradkettiges oder verzweigtes Alkyl mit 1 bis 6 C-Atomen oder für Cycloalkyl mit 3 bis 6 C-Atomen.

Besonders bevorzugt sind 1-Iod-1-propin-3-ole der Formel I, in welcher

$R^1$ für gegebenenfalls durch Fluor, Chlor, Trifluormethoxy, Trifluormethylmercapto, 1,2,4-Triazol-1-yl und Imidazol-1-yl substituiertes Phenyl, und

$R^2$ für Methyl, Ethyl, iso-Propyl, n-Propyl, n-Butyl, sek.-Butyl, tert.-Butyl oder Cyclohexyl steht.

Im einzelnen seien ausser den bei den Herstellungsbeispielen genannten Verbindungen die folgenden Verbindungen der allgemeinen Formel I in Tabelle 1 genannt.

*Tabelle 1*

$$I-C\equiv C-\underset{R^2}{\overset{R^1}{C}}-OH \qquad (I)$$

| $R^1$ | $R^2$ |
|---|---|
| 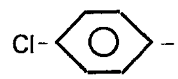 | $-C_2H_5$ |
| 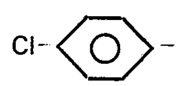 | $-nC_3H_7$ |
| 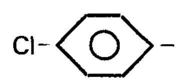 | $-nC_4H_9$ |
| 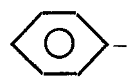 | $-C(CH_3)_3$ |
| 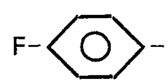 | $-nC_3H_7$ |
| 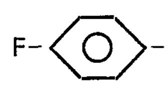 | $-CH_3$ |
| 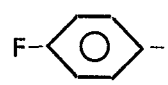 | $-C_2H_5$ |
| 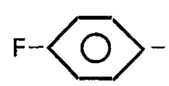 | $-nC_4H_9$ |
| 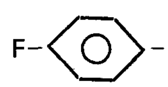 | $-sek.-C_4H_9$ |
| 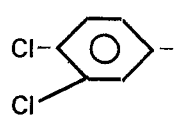 | $-C_2H_5$ |

*Tabelle 1* (Fortsetzung)

| $R^1$ | $R^2$ |
|---|---|
| 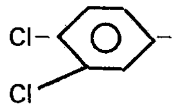 | $-nC_3H_7$ |
| 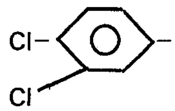 | $-iC_3H_7$ |
| 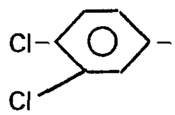 | $-nC_4H_9$ |
| 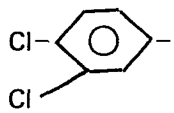 | $-tert.-C_4H_9$ |
| 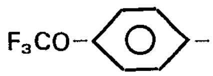 | $-C_2H_5$ |
| 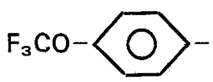 | $-tert.-C_4H_9$ |
| 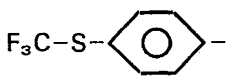 | $-tert.-C_4H_9$ |
| 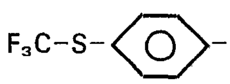 | $-C_2H_5$ |
| 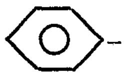 | $-C_2H_5$ |
| 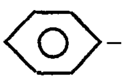 | $-C_3H_7$ |
| 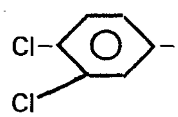 | 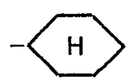 |
| 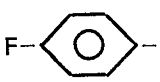 | 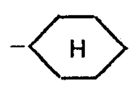 |
| 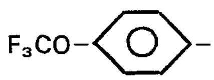 | 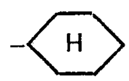 |
| 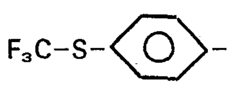 | 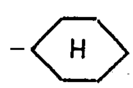 |
| 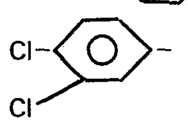 | 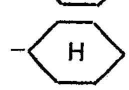 |
| 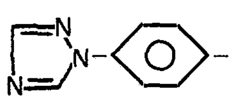 | $-C_2H_5$ |
| 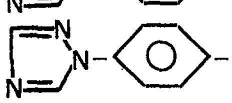 | $-C_3H_7$ |

*Tabelle 1* (Fortsetzung)

| $R^1$ | $R^2$ |
|---|---|
| 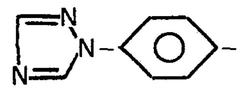 | 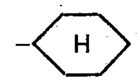 |
| 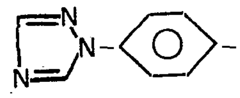 | $-C_2H_5$ |
| 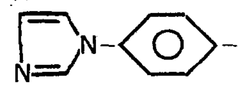 | 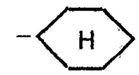 |

Durch diese Tabelle sollen mögliche Kombinationen beliebiger Reste $R^1$ mit beliebigen Resten $R^2$ nicht ausgeschlossen werden, die unter die Definition der allgemeinen Formel I fallen.

Verwendet man beispielsweise 3-(4-Trifluormethoxyphenyl)-1-butin-3-ol und Iod als Ausgangsstoffe, so kann der Reaktionsablauf durch das folgende Formelschema wiedergegeben werden:

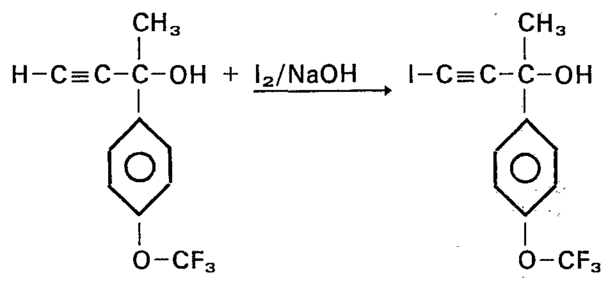


Die bei der Durchführung des erfindungsgemässen Verfahrens als Ausgangsstoffe eingesetzten 1-Propin-3-ole sind durch die Formel II allgemein definiert. In dieser Formel steht vorzugsweise

$R^1$ für gegebenenfalls durch 1,2,4-Triazol-1-yl, Imidazol-1-yl, Fluor, Chlor, Brom, Iod, Halogenalkoxy und Halogenalkylthio mit jeweils 1 oder 2 C-Atomen und 1 bis 5 Halogenatomen substituiertes Aryl mit 6 bis 10 C-Atomen, und

$R^2$ für geradkettiges oder verzweigtes Alkyl mit 1 bis 6 C-Atomen oder für Cycloalkyl mit 3 bis 6 C-Atomen. Besonders bevorzugt sind 1-Iod-1-propin-3-ole der Formel I, in welcher

$R^1$ für gegebenenfalls durch Fluor, Chlor, Trifluormethoxy, Trifluormethylmercapto, 1,2,4-Triazol-1-yl, und Imidazol-1-yl substituiertes Phenyl, und

$R^2$ für Methyl, Ethyl, iso-Propyl, n-Propyl, n-Butyl, sek.-Butyl, tert.-Butyl oder Cyclohexyl steht.

Die 1-Propin-3-ole der Formel II sind bekannt und/oder können nach an sich bekannten Verfahren hergestellt werden (DE-OS Nr. 2438462).

Man erhält sie beispielsweise durch Ethinylierung der entsprechenden Ketone mit Monoalkalimetallsalzen bzw. Mono-Grignard-Verbindungen des Acetylens in geeigneten Lösungsmitteln und nachfolgender Hydrolyse. So können die Verbindungen der Formel II hergestellt werden, indem man das entsprechende Keton in Gegenwart eines Alkalimetallsalzes eines tertiären Alkohols in einem aprotischen Lösungsmittel mit Acetylen umsetzt oder indem man das Keton zu einer aus einem Alkalimetall und Acetylen in flüssigem Ammoniak bereiteten Lösung eines Alkalimetallacetylids zugibt. Weiterhin können die Verbindungen der Formel II beispielsweise durch Umsetzung von Acetylenmagnesiumhalogeniden mit den entsprechenden Ketonen in einem aprotischen Lösungsmittel, beispielsweise Diethylether oder Tetrahydrofuran hergestellt werden. Die Acetylenmagnesiumhalogenide werden dabei durch Einleiten von Acetylen in Lösungen von Alkylmagnesiumhalogeniden erhalten.

Das erfindungsgemässe Verfahren kann in Verdünnungsmitteln ausgeführt werden. Als Verdünnungsmittel können protische Lösungsmittel, beispielsweise Alkohole, wie Methanol, Ethanol, Propanol, Isopropanol und Glykolmonomethylether eingesetzt werden. Gegebenenfalls können auch Mischungen mit anderen Verdünnungsmitteln, wie Wasser oder organischen Aminen, wie Pyridin, Chinolin oder Picolinen eingesetzt werden.

Die erfindungsgemässe Umsetzung wird in Gegenwart einer basischen Verbindung durchgeführt. Bevorzugt werden Erdalkali- und Alkalihydroxide eingesetzt, besonders bevorzugt wässerige Lösungen von Natrium- oder Kaliumhydroxid.

Die Reaktionstemperaturen können beim erfindungsgemässen Verfahren in einem grösseren Bereich variiert werden. Im allgemeinen arbeitet man von 0 bis 50, vorzugsweise von 10 bis 30°C.

Bei der Durchführung des erfindungsgemässen Verfahrens können Iod und die wässerige Alkalihydroxidlösung gleichzeitig zum 1-Propin-3-ol der Formel II gegeben werden. Es kann aber auch das 1-Propin-3-ol der Formel II, Iod und wässerige Alkalihydroxidlösung gleichzeitig zusammengegeben werden.

Die Reaktionspartner werden im allgemeinen in stöchiometrischen Mengen eingesetzt. In besonderen Fällen kann es vorteilhaft sein, einen Reaktionspartner im Überschuss einzusetzen.

Das erfindungsgemässe Verfahren kann wie folgt ausgeführt werden: 1-Propin-3-ol der Formel II wird vorgelegt. Dazu wird die basische Verbindung gegeben und gleichzeitig Iod eingetragen.

Nach Beendigung der Umsetzung wird die Reaktionsmischung mit Wasser versetzt. Die enstandenen 1-Iod-1-propin-3-ole können dann auf übliche Weise isoliert und gegebenenfalls durch Kristallisation oder Destillation gereinigt werden.

Die im erfindungsgemässen Verfahren eingesetzten 1-Propin-3-ole der Formel II können ein asymmetrisches C-Atom enthalten. In besonderen Fällen kann bei der Herstellung der 1-Iodpropin-3-ole der Formel I die Konfiguration dieses C-Atoms beeinflusst werden.

Die erfindungsgemässen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromyzetes, Oomyzetes, Chytridiomyzetes, Zygomyzetes, Ascomyzetes, Basidiomyzetes, Deuteromyzetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemässen Wirkstoffe mit besonders gutem Erfolg zur Bekämpfung von Venturia-Arten, wie gegen den Erreger des Apfelschorfes *(Fusicladium dendriticum)*, verwendet werden; ausserdem auch zur Bekämpfung von Getreidekrankheiten, wie Weizenbraunrost *(Puccinia recondita)*. Die erfindungsgemässen Wirkstoffe besitzen ausserdem gute bakterizide und akarizide Eigenschaften.

Die Wirkstoffe können in die üblichen Formulierungen übergeführt werden, wie Lösungen, Emulsionen, Suspensionen, Pulver, Schäume; Pasten, Granulate, Aerosole, wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebelformulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol, sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosoltreibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Datomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyethylenfettsäureester, Polyoxyethylenfettalkoholether, z.B. Alkylarylpolyglykolether, Alkylsulfonate, Alkylsulfate, Arylsulfonate sowie Eiweisshydrolysate; als Dispergiermittel kommen in Frage: z.B. Ligninsulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90%.

Die erfindungsgemässen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfrass, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Giessen, Tauchen, Spritzen, Sprühen, Vernebeln, Verdampfen, Injizieren, Verschlämmen, Verstreichen, Stäuben, Streuen, Trokkenbeizen, Feuchtbeizen, Nassbeizen, Schlämmbeizen oder Inkrustieren.

Bei der Behandlung von Pflanzenteilen können die Wirkstoffkonzentrationen in den Anwendungsformen in einem grösseren Bereich variiert werden. Sie liegen im allgemeinen zwischen 1 und 0,0001, vorzugsweise zwischen 0,5 und 0,0001 Gew.-%.

Bei der Saatgutbehandlung werden im allgemeinen Wirkstoffmengen von 0,001 bis 50, vorzugsweise 0,01 bis 10 g/kg, benötigt.

Bei Behandlung des Bodens sind Wirkstoffkonzentrationen von 0,00001 bis 0,1, vorzugsweise von 0,0001 bis 0,02 Gew.-%, am Wirkungsort erforderlich.

In entsprechenden Aufwandkonzentrationen zeigen die erfindungsgemässen Wirkstoffe auch bakterizide und akarizide Wirkungen.

*Herstellungsbeispiele*

*Beispiel 1*

*1-Iod-3-(4-trifluormethoxyphenyl)-1-butin-3-ol*

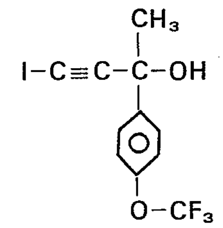

In eine Lösung von 23 g (0,1 mol) 3-(4-Trifluormethoxyphenyl)-1-butin-3-ol in 250 ml Methanol werden bei 15 bis 20°C innerhalb von 30 min 40 ml konzentrierte Natronlauge unter Rühren eingetropft und dabei gleichzeitig portionsweise 25,4 g (0,1 mol) Iod eingetragen. Nach 3 h wird das Reaktionsgemisch in 1000 ml Wasser eingerührt. Das zunächst ölig anfallende Reaktionsprodukt kristallisiert beim Stehen. Der Festkörper wird in 200 ml Essigester aufgenommen, die Lösung zweimal mit je 100 ml Wasser gewaschen und die organische Phase nach dem Trocknen über wasserfreiem Natriumsulfat unter vermindertem Druck eingedampft. Das zurückbleibende Öl wird in wenig Petrolether gelöst, gekühlt und die ausgeschiedenen Kristalle abgesaugt. Man erhält 17,2 g (≙ 48,2% der Theorie) farblose Kristalle vom Schmelzpunkt = Fp.: 75 bis 76°C.

*Herstellung des Ausgangsproduktes*

*3-(4-Trifluormethoxyphenyl)-1-butin-3-ol*

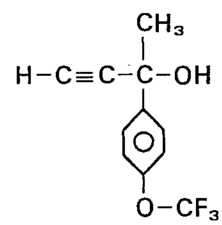

Durch eine Suspension von 47 g (0,42 mol) Kalium-tert.-butylat in 500 ml Tetrahydrofuran wird bei 15°C 30 min ein mittelstarker Acetylenstrom geleitet. Anschliessend wird unter weiterem Einleiten von Acetylen eine Lösung von 62,6 g (0,3 mol) 4-Trifluormethoxyacetophenon in 100 ml Tetrahydrofuran innerhalb 1 h zugetropft. 2 h nach Beendigung der Zugabe des Ketons wird unter Aussenkühlung die Lösung mit 2N-Salzsäure angesäuert, ausgeschiedenes Salz abfiltriert und das Filtrat unter vermindertem Druck eingedampft. Der ölige Rückstand wird in 250 ml Essigester aufgenommen, die organische Phase dreimal mit je 50 ml Wasser gewaschen, über wasserfreiem Natriumsulfat getrocknet und im Vakuum eingedampft. Lösungsmittelreste werden aus dem flüssigen Rückstand bei 50°C bei 0,01 mbar entfernt. Man erhält 61,6 g (89,2% der Theorie) gelbliches Öl vom Brechungsindex $n_D^{20}$ = 1,4659.

In entsprechender Weise werden die nachfolgenden Beispiele der allgemeinen Formel:

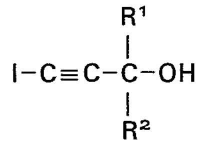 (I)

erhalten:

| Beispiel Nr. | $R^1$ | $R^2$ | Physikalische Konstante |
|---|---|---|---|
| 2 | 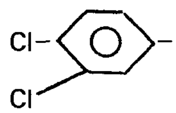 | $-CH_3$ | Fp.: 72-74°C |
| 3 | 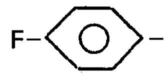 | $-CH(CH_3)_2$ | Fp.: 57-59°C |
| 4 | 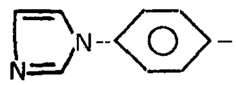 | $-CH_3$ | Fp.: 157-159°C |
| 5 | 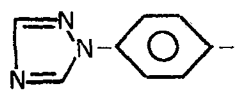 | $-CH_3$ | Fp.: 177-179°C |
| 6 | 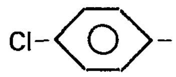 | $-CH(CH_3)_2$ | $n_D^{20} = 1,593$ |
| 7 | 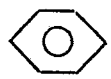 | $-CH_3$ | $n_D^{20} = 1,5661$ |
| 8 | 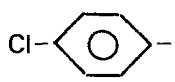 | $-CH_3$ | Fp.: 84-85°C |
| 9 | 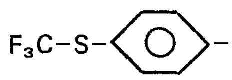 | $-CH_3$ | $n_D^{20} = 1,521$ |
| 10 | 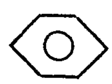 | 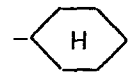 | $n_D^{20} = 1,565$ |

*Verwendungsbeispiele*

In den nachfolgenden Beispielen werden die nachstehend angegebenen Verbindungen als Vergleichssubstanzen eingesetzt

A = 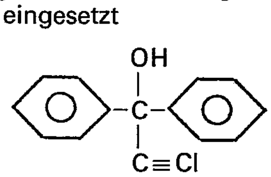

3,3-Diphenyl-1-iod-1-propin-3-ol

B = 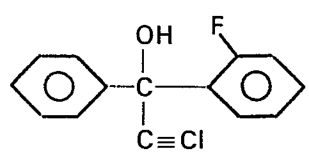

3-(2-Fluorphenyl)-1-iod-3-phenyl-1-propin-3-ol

C = 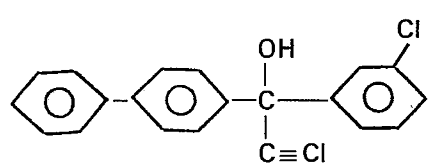

3-(4-Biphenyl-1-yl)-3-(3-chlorphenyl)-1-iod-1-propin-3-ol

*Beispiel A*

Fusicladium-*Test (Apfel) / protektiv*

Lösungsmittel: 4,7 Gew.-Teile Aceton

Emulgator: 0,3 Gew.-Teile Alkylarylpolyglykolether

Wasser: 95,0 Gew.-Teile

Man vermischt die für die gewünschte Wirkstoffkonzentration in der Spritzflüssigkeit nötige Wirkstoffmenge mit der angegebenen Menge Lösungsmittels und verdünnt das Konzentrat mit der

angegebenen Menge Wasser, welches die genannten Zusätze enthält.

Mit der Spritzflüssigkeit bespritzt man junge Apfelsämlinge, die sich im 4- bis 6-Blattstadium befinden, bis zur Tropfnässe. Die Pflanzen verbleiben 24 h bei 20°C und einer relativen Luftfeuchtigkeit von 70% im Gewächshaus. Anschliessend werden sie mit einer wässerigen Konidiensuspension des Apfelschorferregers *(Fusicladium dentriticum)* inokuliert und 18 h lang in einer Feuchtkammer bei 18 bis 20°C und 100% relativer Luftfeuchtigkeit inkubiert.

Die Pflanzen kommen dann erneut für 14 d ins Gewächshaus.

15 d nach der Inokulation wird der Befall der Sämlinge bestimmt. Die erhaltenen Boniturwerte werden in Prozent Befall umgerechnet. 0% bedeutet keinen Befall, 100% bedeutet, dass die Pflanzen vollständig befallen sind.

Eine deutliche Überlegenheit in der Wirksamkeit gegenüber dem Stand der Technik zeigt bei diesem Test z.B. die Verbindung gemäss dem Herstellungsbeispiel 2.

*Beispiel B*

Puccinia-*Test (Weizen) / protektiv*

Lösungsmittel: 100 Gew.-Teile Dimethylformamid

Emulgator: 0,25 Gew.-Teile Alkylarylpolyglykolether

Zur Herstellung einer zweckmässigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von *Puccinia recondita* in einer 0,1%igen wässerigen Agarlösung inokuliert. Nach Antrocknen besprüht man die Pflanzen mit der Wirkstoffzubereitung taufeucht. Die Pflanzen verbleiben 24 h bei 20°C und 100% relativer Luftfeuchtigkeit in einer Inkubationskabine.

Die Pflanzen werden in einem Gewächshaus bei einer Temperatur von ca. 20°C und einer relativen Luftfeuchtigkeit von ca. 80% aufgestellt, um die Entwicklung von Rostpusteln zu begünstigen.

10 d nach der Inokulation erfolgt die Auswertung.

Eine deutliche Überlegenheit in der Wirksamkeit gegenüber dem Stand der Technik zeigen bei diesem Test z.B. die Verbindungen gemäss den Herstellungsbeispielen 2, 8 und 6.

## Patentansprüche

1. 1-Iod-1-propin-3-ole der Formel (I)

$$\begin{array}{c} R^1 \\ | \\ I-C\equiv C-C-OH \\ | \\ R^2 \end{array} \qquad (I)$$

in welcher

$R^1$ für gegebenenfalls durch Azolylreste, Halogen, Halogenalkoxy und Halogenalkylthio substituiertes Aryl, und

$R^2$ für Alkyl oder Cycloalkyl stehen.

2. 1-Iod-1-propin-3-ole gemäss Anspruch 1 und Formel (I), in welcher

$R^1$ für gegebenenfalls durch 1,2,4-Triazol-1-yl, Imidazol-1-yl, Fluor, Chlor, Brom, Jod, Halogenalkoxy und Halogenalkylthio mit jeweils 1 oder 2 C-Atomen und 1 bis 5 Halogenatomen substituiertes Aryl mit 6 bis 10 C-Atomen, und

$R^2$ für geradkettig oder verzweigtes Alkyl mit 1 bis 6 C-Atomen oder für Cycloalkyl mit 3 bis 6 C-Atomen stehen.

3. 1-Iod-1-propin-3-ole gemäss Anspruch 1 und Formel (I), in welcher

$R^1$ für gegebenenfalls durch Fluor, Chlor, Trifluormethoxy, Trifluormethylmercapto, 1,2,4-Triazol-1-yl und Imidazol-1-yl substituiertes Phenyl, und

$R^2$ für Methyl, Ethyl, iso-Propyl, n-Propyl, n-Butyl, sek.-Butyl, tert.-Butyl oder für Cyclohexyl stehen.

4. Verfahren zur Herstellung von 1-Iod-1-propin-3-olen der Formel (I)

$$\begin{array}{c} R^1 \\ | \\ I-C\equiv C-C-OH \\ | \\ R^2 \end{array} \qquad (I)$$

in welcher

$R^1$ für gegebenenfalls durch Azolylreste, Halogen, Halogenalkoxy und Halogenalkylmercapto substituiertes Aryl, und

$R^2$ für Alkyl oder Cycloalkyl steht,

dadurch gekennzeichnet, dass man 1-Propin-3-ole der Formel (II)

$$\begin{array}{c} R^1 \\ | \\ H-C\equiv C-C-OH \\ | \\ R^2 \end{array} \qquad (II)$$

in welcher

$R^1$ und $R^2$ die in Anspruch 1 angegebene Bedeutung haben,

mit Iod in Gegenwart einer basischen Verbindung und gegebenenfalls in Gegenwart eines Verdünnungsmittels umsetzt.

5. Verfahren nach Anspruch 4, dadurch gekennzeichnet, dass in Gegenwart eines Verdünnungsmittels umgesetzt wird.

6. Verfahren nach Anspruch 4, dadurch gekennzeichnet, dass bei einer Temperatur von 0 bis 50°C umgesetzt wird.

7. Fungizide Mittel, gekennzeichnet durch einen Gehalt an mindestens einem 1-Iod-1-propin-3-ol der Formel (I).

8. Verwendung von 1-Iod-1-propin-3-olen der Formel (I) zur Bekämpfung von pflanzenpathogenen Pilzen.

9. Verfahren zur Bekämpfung von pflanzenpathogenen Pilzen, dadurch gekennzeichnet, dass man 1-Iod-1-propin-3-ole der Formel (I) auf

pflanzenpathogene Pilze und/oder ihren Lebensraum einwirken lässt.

10. Verfahren zur Herstellung fungizider Mittel, dadurch gekennzeichnet, dass man 1-Iod-1-propin-3-ole der Formel (I) mit Streckmitteln und/oder oberflächenaktiven Mitteln vermischt.

**Claims**

1. 1-Iodoprop-1-yn-3-ols of the Formula (I)

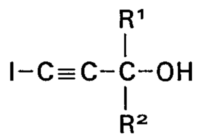 (I)

in which

$R^1$ represents aryl which is optionally substituted by azolyl radicals, halogen, halogenoalkoxy or halogenoalkylthio, and

$R^2$ represents alkyl or cycloalkyl.

2. 1-Iodoprop-1-yn-3-ols according to Claim 1 and Formula (I), in which

$R^1$ represents aryl which has 6 to 10 C atoms and is optionally substituted by 1,2,4-triazol-1-yl-imidazol-1-yl, fluorine, chlorine, bromine, iodine, or halogenoalkoxy or halogenoalkylthio with in each case 1 or 2 C atoms and 1 to 5 halogen atoms, and

$R^2$ represents straight-chain or branched alkyl with 1 to 6 C atoms or cycloalkyl with 3 to 6 C atoms.

3. 1-Iodoprop-1-yn-3-ols according to Claim 1 and Formula (I), in which

$R^1$ represents phenyl which is optionally substituted by fluorine, chlorine, trifluoromethoxy, trifluoromethylmercapto, 1,2,4-triazol-1-yl or imidazol-1-yl, and

$R^2$ represents methyl, ethyl, iso-propyl, n-propyl, n-butyl, sec.-butyl, tert.-butyl or cyclohexyl.

4. Process for the preparation of 1-iodoprop-1-yn-3-ols of the Formula (I)

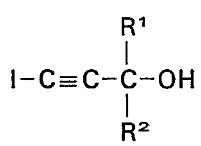 (I)

in which

$R^1$ represents aryl which is optionally substituted by azolyl radicals, halogen, halogenoalkoxy or halogenoalkylmercapto, and

$R^2$ represents alkyl or cycloalkyl,

characterised in that prop-1-yn-3-ols of the Formula (II)

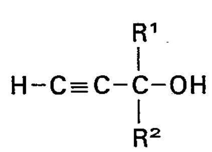 (II)

in which

$R^1$ and $R^2$ have the meaning indicated in Claim 1, are reacted with iodine in the presence of a basic compound and, if appropriate, in the presence of a diluent.

5. Process according to Claim 4, characterised in that the reaction is carried out in the presence of a diluent.

6. Process according to Claim 4, characterised in that the reaction is carried out at a temperature of 0 to 50°C.

7. Fungicidal agents, characterised in that they contain at least one 1-iodoprop-1-yn-3-ol of the Formula (I).

8. Use of 1-iodoprop-1-yn-3-ols of the Formula (I) for combating plant-pathogenic fungi.

9. Process for combating plant-pathogenic fungi, characterised in that 1-iodoprop-1-yn-3-ols of the Formula (I) are allowed to act on plant-pathogenic fungi and/or their habitat.

10. Process for the preparation of fungicidal agents, characterised in that 1-iodoprop-1-yn-3-ols of the Formula (I) are mixed with extenders and/or surface-active agents.

**Revendications**

1. 1-iodo-1-propin-3-ols de formule (I):

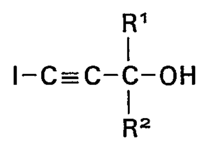 (I)

dans laquelle

$R^1$ représente un groupe aryle éventuellement substitué par un radical azolyle, par un atome d'halogène, par un groupe halogénoalcoxy et par un groupe halogénoalkylthio, et

$R^2$ représente un groupe alkyle ou un groupe cycloalkyle.

2. 1-iodo-1-propin-3-ols suivant la revendication 1 et répondant à la formule (I) dans laquelle

$R^1$ représente un groupe aryle contenant 6 à 10 atomes de carbone et éventuellement substitué par un groupe 1,2,4-triazol-1-yle, par un groupe imidazol-1-yle, par un atome de fluor, par un atome de chlore, par un atome de brome, par un atome d'iode, par un groupe halogénoalcoxy et par un groupe halogénoalkylthio contenant chacun 1 ou 2 atomes de carbone et 1 à 5 atomes d'halogènes, et

$R^2$ représente un groupe alkyle à chaîne droite ou ramifiée contenant 1 à 6 atomes de carbone ou un groupe cycloalkyle contenant 3 à 6 atomes de carbone.

3. 1-iodo-1-propin-3-ols suivant la revendication 1 et répondant à la formule (I) dans laquelle

$R^1$ représente un groupe phényle éventuellement substitué par un atome de fluor, par un atome de chlore, par un groupe trifluorométhoxy, par un groupe trifluorométhylmercapto, par un groupe 1,2,4-triazol-1-yle et par un groupe imidazol-1-yle, et

$R^2$ représente un groupe méthyle, un groupe éthyle, un groupe isopropyle, un groupe n-propyle, un groupe n-butyle, un groupe sec.-

butyle, un groupe tert.-butyle ou un groupe cyclohexyle.

4. Procédé de préparation de 1-iodo-1-propin-3-ols de formule (I):

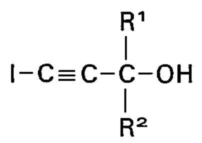

(I)

dans laquelle

$R^1$ représente un groupe aryle éventuellement substitué par un radical azolyle, par un atome d'halogène, par un groupe halogénoalcoxy et par un groupe halogénoalkylmercapto, et

$R^2$ représente un groupe alkyle ou un groupe cycloalkyle,

caractérisé en ce qu'on fait réagir des 1-propin-3-ols de formule (II):

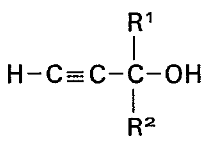

(II)

dans laquelle

$R^1$ et $R^2$ ont les significations indiquées dans la revendication 1,

avec de l'iode en présence d'un composé basique et éventuellement en présence d'un diluant.

5. Procédé suivant la revendication 4, caractérisé en ce qu'on fait réagir en présence d'un diluant.

6. Procédé suivant la revendication 4, caractérisé en ce qu'on fait réagir à une température de 0 à 50°C.

7. Agents fongicides, caractérisés en ce qu'ils contiennent au moins un 1-iodo-1-propin-3-ol de formule (I).

8. Utilisation de 1-iodo-1-propin-3-ols de formule (I) pour combattre les champignons pathogènes des plantes.

9. Procédé en vue de combattre les champignons pathogènes des plantes, caractérisé en ce qu'on fait agir des 1-iodo-1-propin-3-ols de formule (I) sur des champignons pathogènes des plantes et/ou leur biotope.

10. Procédé de préparation d'agents fongicides, caractérisé en ce qu'on mélange des 1-iodo-1-propin-3-ols de formule (I) avec des diluants et/ou des agents tensio-actifs.